Europäisches Patentamt

European Patent Office

Office européen des brevets

(11) Veröffentlichungsnummer : **0 125 513**
**B1**

(12) **EUROPÄISCHE PATENTSCHRIFT**

(45) Veröffentlichungstag der Patentschrift :
09.09.87

(51) Int. Cl.⁴ : **G 01 N 33/72, C 12 Q  1/28**

(21) Anmeldenummer : **84104240.1**

(22) Anmeldetag : **14.04.84**

(54) **Verfahren und Reagens zur Bestimmung des Hämoglobin-Haptoglobin-Komplexes in Gegenwart von freiem Hämoglobin.**

(30) Priorität : **20.04.83 DE 3314308**

(43) Veröffentlichungstag der Anmeldung :
**21.11.84 Patentblatt 84/47**

(45) Bekanntmachung des Hinweises auf die Patenterteilung : **09.09.87 Patentblatt 87/37**

(84) Benannte Vertragsstaaten :
**AT BE CH DE FR GB IT LI LU NL SE**

(56) Entgegenhaltungen :
**DE-A- 3 141 146**
**BIOCHIMICA ET BIOPHYSICA ACTA, Band 285, 1972**
**K.Kawamura et al. "Kinetics of peroxidase activity, absorption spectra and oxygen affinity of human hemoglobin-haptoglobin i-i complexes"**

(73) Patentinhaber : **Boehringer Mannheim GmbH**
**Sandhofer Strasse 116**
**D-6800 Mannheim 31 (DE)**

(72) Erfinder : **Schmitt, Urban**
**Kustermannstrasse 29**
**D-8132 Tutzing (DE)**
Erfinder : **Deeg, Rolf, Dr. rer. nat.**
**Seeseitener Strasse 18**
**D-8124 Seeshaupt (DE)**
Erfinder : **Ziegenhorn, Joachim, Dr. rer. nat.**
**Ina-Seidel-Weg 1**
**D-8130 Starnberg (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Hämoglobin besitzt peroxidatische Aktivität, die auch im Hämoglobin-Haptoglobin-Komplex erhalten bleibt. Die Bestimmung von Hämoglobin über seine Peroxidase-Aktivität ist wesentlich empfindlicher als die direkte photometrische Bestimmung von geeigneten Hämoglobin-Derivaten, beispielsweise von Cyan-Methämoglobin. Die peroxidatische Aktivität von freiem Hämoglobin und von dem Hämoglobin-Haptoglobin-Komplex läßt sich unterschiedlich beeinflussen, beispielsweise durch Verändern des pH-Wertes.

Zur Messung der peroxidatischen Aktivität von freiem Hämoglobin und von dem Hämoglobin-Haptoglobin-Komplex sind zwei verschiedene Meßprinzipien beschrieben (vgl. hierzu : Z. Klin. Chem. u. Klin. Biochem. 6 (1968) S. 69 bis 112 sowie Biochim. Biophys. Acta 285 (1972) S. 22 bis 27) :

a) Die zu bestimmende, Hämoglobin- bzw. den Hämoglobin-Haptoglobin-Komplex enthaltende Lösung wird mit Wasserstoffperoxid und mit einem geeigneten chromogenen Substrat versetzt und bis zum Ende der Farbentwicklung inkubiert. Das Ende der Reaktion kommt dadurch zustande, daß das Hämoglobin durch das Wasserstoffperoxid inaktiviert wird. Die maximal erreichte Extinktion wird gemessen. Oft nimmt die Extinktion nach Erreichen dieses Maximums infolge Instabilität des gebildeten Farbstoffs wieder ab.

b) Die Reaktionsgeschwindigkeit der Umsetzung zwischen Hämoglobin bzw. Hämoglobin-Haptoglobin-Komplex, Wasserstoffperoxid und chromogenem Substrat wird zu Beginn der Reaktion gemessen (Anfangsgeschwindigkeit).

Nachteile der Methode a) bei der Bestimmung des Hämoglobin-Haptoglobin-Komplexe sind u. a., daß sich für den Komplex ein nicht linearer Zusammenhang zwischen Meßwert und Konzentration ergibt und das freie Hämoglobin in Abhängigkeit von der eingesetzten Wasserstoffperoxid-Konzentration einmal eine höhere, einmal eine niedrigere Aktivität als der Komplex zeigt.

Bei der Bestimmung der Komplex-Konzentration in Gegenwart von freiem Hämoglobin ist die Methode b) der Methode a) vorzuziehen, da bei letzterer ein linearer Zusammenhang zwischen Meßwert und Konzentration an Hämoglobin-Haptoglobin-Komplex besteht und bei Verschiebung des pH-Wertes in den sauren Bereich, vorzugsweise auf einen pH-Wert von ungefähr 4, die peroxidatische Aktivität des Komplexes immer höher ist als diejenige des freien Hämoglobins.

Die Genauigkeit der Messung hängt entscheidend von der Differenzierung der peroxidatischen Aktivität von Hämoglobin in freier und gebundener Form ab. Bei den bisher beschriebenen Bestimmungsmethoden hat man im wesentlichen von der unterschiedlichen Beeinflussung der peroxidatischen Aktivität durch Veränderung des pH-Wertes Gebrauch gemacht. Das Verhältnis der Aktivitäten gleicher molarer Mengen Komplex und freiem Hämoglobin liegt bei pH-Werten um 4 bei 10 : 1 bis 20 : 1. Es hängt vom verwendeten chromogenen Substrat und von der eingesetzten Substrat- und der Hämoglobin-Konzentration ab. Die stets noch vorhandene Restaktivität des freien Hämoglobins verhindert eine zuverlässige und exakte Messung der Konzentration an Hämoglobin-Haptoglobin-Komplex, vor allem dann, wenn mehr freies Hämoglobin als im Komplex gebundenes Hämoglobin vorliegt.

Aufgabe der vorliegenden Erfindung war es, ein Verfahren zur Bestimmung von Hämoglobin-Haptoglobin-Komplex in Gegenwart von freiem Hämoglobin bereitzustellen, bei dem die Peroxidase-Aktivität des freien Hämoglobins weitgehend inaktiviert wird und die Peroxidase-Aktivität des Hämoglobin-Haptoglobin-Komplexes ungestört und somit genau erfaßt werden kann.

Gelöst wird diese Aufgabe dadurch, daß dem Reaktionsgemisch ein Detergens aus der Gruppe der Saponine, Lanoline und Detergentien mit einem Zuckeranteil zugesetzt wird, das die Peroxidase-Aktivität des freien Hämoglobins nahezu vollständig inaktiviert, die peroxidatische Aktivität des im Komplex gebundenen Hämoglobins jedoch nicht oder nur unwesentlich verändert.

Gegenstand der Erfindung ist der in den Ansprüchen näher gekennzeichnete Gegenstand.

Die erfindungsgemäß verwendbaren Detergentien inaktivieren weitgehend die Peroxidase-Aktivität des freien Hämoglobins, ohne die Peroxidase-Aktivität des gebundenen Hämoglobins merklich zu beeinflussen und unterbinden auch nicht die peroxidatische Farbstoffbildung. Besonders bevorzugt im Sinne der Erfindung sind Saponin, Digitonin und Polyoxyethylen-Sorbit, beispielsweise das Detergens G 2330® der Firma Atlas.

Unter Saponinen wird eine Gruppe von im allgemeinen pflanzlichen Glykosiden verstanden, die in Wasser kolloidale, seifenartige Lösungen bilden. Die Aglykone dieser Glykoside besitzen Triterpenoid- oder Steroid-Strukturen. Das Digitonin, ein Digitalis-Glykosid, sowie das handelsübliche Saponin, die im allgemeinen aus Saponinen bestehenden Inhaltsstoffe der Seifenwurzel und der Panamarinde, gehören zu dieser Gruppe von Detergentien.

Die Lanoline stellen kompliziert aufgebaute Estergemische aus Fettsäuren, wie Palmitinsäure, Capronsäure, Oelsäure u. a., und aliphatischen, Triterpenoid- oder Steroid-Alkoholen, wie beispielsweise Cetylalkohol, Cholesterin, Lanosterin u. a., dar. Diese Estergemische reichern sich in der Schafwolle an und werden daraus gewonnen.

Die peroxidatische Aktivität kann auch nach Zusatz eines erfindungsgemäßen Detergens in bekannter Weise bestimmt werden. Hierzu stehen dem Fachmann eine Reihe von Verfahren zur Verfügung. Im allgemeinen wird ein Peroxid der zu bestimmenden Lösung zugesetzt und ein Substrat hinzugegeben, das unter der Einwirkung der Peroxidase und dem Peroxid eine charakteristische, leicht meßbare Farbänderung erfährt.

Zur Bestimmung des Hämoglobin-Haptoglobin-Komplexes in Gegenwart von freiem Hämoglobin wird vorzugsweise der den Komplex und das freie Hämoglobin enthaltenden Probe das Detergens, das chromogene Substrat, ein geeignetes Puffersystem sowie gegebenenfalls weitere Hilfsstoffe zugesetzt, die Peroxidase-Reaktion durch Zugabe eines Peroxids gestartet und die Extinktionsänderung photometrisch erfaßt. Die Reaktionsgeschwindigkeit wird ausgedrückt als Extinktionsänderung pro Zeiteinheit ($\Delta$E/min).

Die Konzentration des Detergens kann in weiten Grenzen frei gewählt werden. Die eingesetzte Menge hängt im wesentlichen davon ab, welches Detergens verwendet wird und welche Differenzierung der peroxidatischen Aktivität erreicht werden soll, um mit dem gewählten Peroxidase-Nachweissystem einen brauchbaren, genauen Meßwert zu erhalten. Nach oben wird die einsetzbare Konzentration an Detergens durch dessen Löslichkeit in der Reaktionslösung begrenzt. Wird diese maximale Löslichkeit überschritten, so treten Trübungen auf, die das Bestimmungsverfahren beeinträchtigen bzw. unmöglich machen.

Als chromogene Substrate kommen diejenigen infrage, die sich von Peroxidasen in sauren Milieu mit Peroxid umsetzen lassen. Insbesondere haben sich bewährt Guajakol, 2,2'-Azino-di-[3-äthylbenzthiazolinsulfonsäure(6)] (ABTS) sowie die Zwei-Komponenten-Systeme 4-Aminoantipyrin (AAP), 3-Methyl-2-benzthiazolonhydrazon (MBTH) oder 3-Methyl-2-benzthiazolonhydrazonsulfonsäure (SMBTH) in Verbindung mit einem Phenol- oder Anilin-Derivat (vgl. Analytical Chemistry 33 (1961) S. 722 bis 725). Das chromogene Substrat wird üblicherweise in einer Konzentration von 0,1 bis 50,0 mmol/l eingesetzt. Sie hängt wesentlich von dem gewählten Substrat ab. So liegt der bevorzugte Konzentrationsbereich für ABTS und AAP bei 0,1 bis 5 mmol/l, für Guajakol bei 1 bis 50 mmol/l und für MBTH und SMBTH bei 0,1 bis 1 mmol/l. Die Phenol- bzw. Anilin-Derivate werden bei den Zwei-Komponenten-Systemen in einer ungefähr 10-fach höheren Konzentration als die entsprechenden Kupplungspartner eingesetzt.

Als Puffersystem kommen alle Puffer infrage, deren Pufferwirkung im sauren pH-Bereich liegt, beispielsweise Acetat-Puffer, Citratpuffer u. a. Im Sinne der Erfindung besonders geeignet sind Citratpuffer mit einer Pufferwirkung im Bereich pH 3,5 bis 4,5, besonders bevorzugt ist ein Citratpuffer, pH 3,8 bis 4,0. Die Pufferkonzentration wird in dem Bereich von 0,001 bis 0,5 mol/l gewählt. Bevorzugt liegt sie bei 0,01 bis 0,2 mol/l.

Als Peroxid wird vorzugsweise Wasserstoffperoxid oder Perborat eingesetzt. Die Konzentration des Peroxids in der Testlösung sollte bei 0,1 bis 100, vorzugsweise 0,5 bis 25 mmol/l liegen.

Das Bestimmungsverfahren wird üblicherweise bei einer Temperatur von 20 bis 45 °C, vorzugsweise von 25 bis 30 °C durchgeführt.

Die zu bestimmende Probe sollte eine meßbare Hämoglobin-Konzentration von $0,5 \times 10^{-8}$ bis $2 \times 10^{-7}$ mol/l aufweisen.

Ein weiterer Gegenstand der Erfindung ist ein Reagens zur Bestimmung des Hämoglobin-Haptoglobin-Komplexes in Gegenwart von freiem Hämoglobin, das neben der üblicherweise zur Bestimmung der peroxidatischen Aktivität erforderlichen Substanzen ein Detergens aus der Gruppe der Saponine, Lanoline und Detergentien mit einem Zuckeranteil enthält, das die Peroxidase-Aktivität des freien Hämoglobins inhibiert. Vorzugsweise enthält ein solches Reagens als Substanzen zum Nachweis der peroxidatischen Aktivität ein Peroxid, ein chromogenes Substrat, ein geeignetes Puffersystem und gegebenenfalls weitere Hilfsmittel.

Ein erfindungsgemäß besonders geeignetes Reagens enthält die genannten Bestandteile in den folgenden Konzentrationen :

0,1-50 mg/l Detergens
0,1-100 mmol/l Peroxid
0,1-5 mmol/l chromogenes Substrat
0,001-0,5 mol/l Puffer.

Ganz besonders bevorzugt ist ein Reagens der folgenden Zusammensetzung :

1-5 g/l Saponin
0,5-25 mmol/l Perborat
0,5-2 mmol/l ABTS
0,01-0,2 mol/l Citratpuffer, pH 3,5-4,5.

Das erfindungsgemäße Verfahren läßt sich in vorteilhafter Weise auch ausnützen zur Bestimmung von Haptoglobin, indem das Haptoglobin durch Zugabe eines Überschusses an Hämoglobin vollständig in den Hämoglobin-Haptoglobin-Komplex überführt und die Konzentration des Komplexes nach dem erfindungsgemäßen Verfahren gemessen wird. Die zugesetzte Hämoglobin-Menge wird durch das Haptoglobin zu einem Haptoglobin-Hämoglobin-Komplex gebunden bis die gesamte, in der Probe vorhandene Haptoglobin-Menge als Komplex vorliegt. Das überschüssige Hämoglobin bleibt als freies Hämoglobin in Lösung. Dieses trägt bei der Messung der peroxidatischen Aktivität nach dem erfindungsgemäßen Verfahren nicht zu einer Extinktionssteigerung bei, da ja die Peroxidase-Aktivität des freien Hämoglobins durch das zugesetzte Detergens weitgehend inaktiviert wird. Die gemessene Extinktion entspricht der Konzentration an Haptoglobin-Hämoglobin-Komplex und damit der in der Probe ursprünglich vorhandenen Haptoglobin-Konzentration.

Das erfindungsgemäße Verfahren zur Messung des Haptoglobin-Hämoglobin-Komplexes in Gegenwart von freiem Hämoglobin kann auch in vorteilhafter Weise zur Bestimmung des glykosilierten Hämoglobin-Anteils gemäß den in der deutschen Patentanmeldung DE-A-3 141 146 beschriebenen und beanspruchten Verfahren ausgenutzt werden. Hierzu wird die zu bestimmende Probe zur Differenzierung des glykosilierten und des nicht-glykosilierten Anteils mit Haptoglobin versetzt.

Haptoglobin bildet mit glykosiliertem Hämoglobin schneller einen Komplex als mit dem nicht-glykosilierten Hämoglobin. Der glykosilierte Hämoglobin-Haptoglobin-Komplex kann aufgrund seiner peroxidatischen Aktivität nachgewiesen werden.

Normalerweise beträgt die Konzentration an glykosiliertem Hämoglobin im Blut 5 %. Bei Diabetikern ist dieser Anteil auf bis zu 20 % erhöht. In einer mit Haptoglobin versetzten Probe liegt somit der glykosilierte Hämoglobin-Haptoglobin-Komplex neben einem großen Überschuß an freiem Hämoglobin vor. Hier bewährt sich ganz besonders der erfindungsgemäße Zusatz eines Detergens, das die Peroxidase-Aktivität des freien Hämoglobins inaktiviert, diejenige des an Haptoglobin gebundenen Anteils jedoch unbeeinflußt läßt. Auf diese Weise ist es möglich, die peroxidatische Aktivität des glykosilierten Hämoglobin-Haptoglobin-Komplexes hinreichend exakt zu bestimmen, ohne das freie Hämoglobin in der Probe abtrennen zu müssen.

Eine vorteilhafte Ausführungsform dieses Bestimmungsverfahrens besteht darin, daß man aus der Probe, beispielsweise Vollblut, die Erythrozyten abtrennt und hämolysiert, das Hämolysat mit Dithionit bis zu einer Konzentration von 10 bis 100 mg/ml und mit Nitrit bis zu einer Konzentration von 0,2 bis 10 mg/ml versetzt und mit einer Pufferlösung (pH 5,5 bis 7,0) bzw. mit einer Pufferlösung (pH 5,5 bis 7,0), die eine Verbindung mit Bindungswirkung auf die allosterischen Effektorstellen des Hämoglobins, beispielsweise Inosithexaphosphat, 2,3-Diphosphoglycerat u. a., in einer Konzentration von 0,04 bis 0,2 mmol/l enthält, auf eine Hämoglobin-Konzentration von ungefähr 0,5 mg/l einstellt. Ein Volumteil dieser Hämoglobin-Probe wird mit einem Volumteil einer Reagentienlösung versetzt, die 5 bis 50 mmol/l Puffer, pH 5,5 bis 7,0, 10 bis 50 mg/l Haptoglobin, 0,1 bis 0,4 g/l Saccharose und 0,2 bis 50,0 mmol/l chromogenes Substrat enthält. Die Peroxidasereaktion wird durch Zusatz einer Lösung gestartet, die das Peroxid und das erfindungsgemäße Detergens enthält. Die Peroxidasereaktion wird durch Messen der Extinktionszunahme bei der für das eingesetzte chromogene Substrat charakteristischen Wellenlänge verfolgt. Es wird die Reaktionsgeschwindigkeit, ausgedrückt in mE/min, bestimmt.

Das vorstehend beschriebene Bestimmungsverfahren eignet sich auch besonders gut zur Durchführung auf einem automatischen Analysenautomaten.

Die vorliegende Erfindung wird durch die folgenden Beispiele näher erläutert :

## Beispiel 1

2 ml einer Reagentienlösung mit 0,1 mol/l Natriumcitratpuffer, pH 3,8, und 1 mmol/l ABTS werden mit 0,025 ml einer Probelösung versetzt, die freies Hämoglobin oder den Hämoglobin-Haptoglobin-Komplex enthält, wobei die Hämoglobin-Konzentration in der Probe jeweils 0,25 mg Hämoglobin/ml beträgt. Die Peroxidase-Reaktion wird durch Zugabe von 0,020 ml 0,58-molarer Natriumperborat-Lösung in 0,15-molarer Citronensäure gestartet und die Farbstoffentwicklung durch Extinktionsmessung bei 436 nm verfolgt (Eppendorf-Photometer mit angeschlossenem Schreiber). Die Reaktionstemperatur beträgt 25 °C.

Gleiche Messungen werden durchgeführt mit Reagenslösungen, die neben dem oben aufgeführten Natriumcitratpuffer und dem ABTS zusätzlich Saponin in steigenden Konzentrationen enthalten. Die für freies Hämoglobin und für den Hämoglobin-Haptoglobin-Komplex ohne Detergenszusatz und bei verschiedenen Saponin-Konzentrationen gemessene Reaktionsgeschwindigkeiten ($\Delta$E/min) sind in Tabelle 1 wiedergegeben.

## Tabelle 1

Reaktionsgeschwindigkeit ($\Delta$E/min) für die Peroxidase-Reaktion von freiem Hämoglobin bzw. Hämoglobin-Haptoglobin-Komplex mit Perborat und ABTS als Substrat bei verschiedenen Saponin-Konzentrationen

(Siehe Tabelle Seite 5 f.)

| ABTS (mmol/l) | Saponin (mg/ml) | ΔE/min | | Hb : Hp |
|---|---|---|---|---|
| | | Hb (mE/min) | Hb : Hp (mE/min) | Hb |
| 1 | — | 172 | 968 | 5,6 |
| 1 | 0,5 | 75 | 1 003 | 13,4 |
| 1 | 1,0 | 57 | 1 003 | 17,4 |
| 1 | 2,0 | 48 | 968 | 20,4 |
| 1 | 3,0 | 40 | 932 | 23,3 |
| 1 | 4,0 | 30 | 1 003 | 33,4 |
| 1 | 5,0 | 15 | 1 003 | 66 |

Ohne Zusatz von Saponin ist bei pH-Wert 3,8 die Reaktivität des freien Hämoglobins lediglich um den Faktor 1 : 5,6 geringer als die Reaktivität des Komplexes. Der Tabelle 1 kann entnommen werden, daß durch steigende Saponin-Zusätze die peroxidatische Aktivität des freien Hämoglobins deutlich gesenkt wird, während sich die Aktivität des Komplexes nicht merklich ändert. Wird Saponin in einer Menge von 5 mg/ml zugegeben, so ist die Aktivität des freien Hämoglobins auf 1/10 des Ausgangswertes gesunken. Das Verhältnis der peroxidatischen Aktivität des freien Hämoglobins zu dem Komplex beträgt dann 1 : 66.

## Beispiel 2

2 ml Reagens mit 0,1 mol/l Natriumcitratpuffer, pH 3,8, und 0,5 mmol/l ABTS werden mit 0,025 ml einer Probe versetzt, die freies Hämoglobin oder Hämoglobin-Haptoglobin-Komplex mit einer Hämoglobin-Konzentration von 0,25 mg Hämoglobin/ml enthält. Es wird, wie in Beispiel 1 angegeben, weitergearbeitet. Analoge Messungen werden durchgeführt mit Reagenslösungen, denen Saponin in unterschiedlichen Konzentrationen zugesetzt worden waren. In Tabelle 2 sind die gefundenen Meßwerte zusammengestellt.

## Tabelle 2

Reaktionsgeschwindigkeit (ΔE/min) der Peroxidase-Reaktion zwischen freiem Hämoglobin bzw. dem Hämoglobin-Haptoglobin-Komplex mit Perborat und ABTS als Substrat in Gegenwart verschiedener Saponin-Konzentrationen

| ABTS (mmol/l) | Saponin (mg/ml) | ΔE/min | | Hb : Hp |
|---|---|---|---|---|
| | | Hb (mE/min) | Hb : Hp (mE/min) | Hb |
| 0,5 | — | 78,8 | 1 285 | 16,3 |
| 0,5 | 0,5 | 32 [1] | 1 175 | 70 |
| 0,5 | 1,0 | 7,5 | 1 175 | 150 |
| 0,5 | 3,0 | 12,5[2] | 1 175 | 375 |
| 0,5 | 5,0 | 30 [3] | 873 | 230 |

[1] Hämoglobin-Konzentration 0,50 mg Hb/ml
[2] Hämoglobin-Konzentration 1,00 mg Hb/ml
[3] Hämoglobin-Konzentration 2,00 mg Hb/ml

Die in Tabelle 2 angegebenen Werte zeigen, daß bei niedrigerer ABTS-Konzentration die Reaktivitätsunterschiede zwischen freiem Hämoglobin und Hämoglobin-Haptoglobin-Komplex ohne Zusatz eines Detergens größer sind. Durch Zusatz von Saponin wird die peroxidatische Aktivität des freien Hämoglobins nahezu vollständig inaktiviert. Um noch gut meßbare Werte zu erhalten, müssen die Hämoglogin-Konzentrationen bis zum achtfachen Wert gegenüber den in Beispiel 1 eingesetzten Konzentrationen erhöht werden.

## Beispiel 3

2 ml einer Reagenslösung mit 0,1 mol/l Natriumcitratpuffer, pH 3,8, und 0,5 mmol/l ABTS werden mit 0,025 ml einer Probelösung versetzt, die freies Hämoglobin oder den Hämoglobin-Haptoglobin-Komplex mit einer Hämoglobin-Konzentration von 0,25 mg Hb/ml enthält. Die Peroxidase-Reaktion wird mit

**0 125 513**

0,020 ml 0,58-molaren Natriumperborat-Lösung in 0,15-molarer Citronensäure gestartet und die Farbstoffentwicklung durch Extinktionsmessung bei 436 nm verfolgt. Die Reaktionstemperatur beträgt 25 °C.

Analoge Messungen werden durchgeführt, wobei der Reagenslösung zusätzlich zu den oben angeführten Bestandteilen die in Tabelle 3 angegebenen Detergentien in den dort ebenfalls angegebenen Konzentrationen zugesetzt werden. Die Meßwerte sind in Tabelle 3 zusammengefaßt.

### Tabelle 3

Reaktionsgeschwindigkeiten ($\Delta$E/min) der Peroxidase-Reaktion für freies Hämoglobin und den Hämoglobin-Haptoglobin-Komplex mit Peroxid und ABTS als Substrat in Gegenwart verschiedener Detergentien.

| ABTS (mmol/l) | Detergens (mg/ml) | $\Delta$E/min | | $\dfrac{\text{Hb : Hp}}{\text{Hb}}$ |
|---|---|---|---|---|
| | | Hb (mE/min) | Hb : Hp (mE/min) | Hb |
| 0,5 | — | 55 | 1 002 | 18,2 |
| 0,5 | 0,15 Digitonin | 21,8 | 818 | 37,6 |
| 0,5 | 0,3 Digitonin | 20 | 818 | 41 |
| 0,5 | 20 G2330[1] | 8,8 | 845 | 97 |
| 0,5 | 40 G2330[1] | 0-5 | 873 | >100 |

[1] G 2330 = Polyoxyethylen-Sorbit-Detergens der Fa. Atlas

### Beispiel 4

2 ml einer Reagenslösung mit 0,1 mol/l Natriumacetatpuffer, pH 4,0, und 30 bzw. 15 mmol/l Guajakol werden mit 0,05 ml einer Probelösung versetzt, die freies Hämoglobin oder den Hämoglobin-Haptoglobin-Komplex mit einer Hämoglobin-Konzentration von 0,25 mg Hb/ml enthält. Die Peroxidase-Reaktion wird mit 0,1 ml 0,58-molarer Natriumperboratlösung in 0,15-molarer Citronensäure gestartet und die Farbstoffentwicklung durch Extinktionsmessung bei 436 nm verfolgt. Die Reaktionstemperatur beträgt 25 °C. Die Farbentwicklung kommt nach einer gewissen Zeit zum Stehen. Die Extinktion nimmt infolge der Instabilität des Farbstoffs wieder ab. Die in der folgenden Tabelle 4 angegebenen Extinktionsänderungen sind die durch Anlegen der Tangente an die Extinktionszeit-Kurven bestimmten Anfangsgeschwindigkeiten.

Analoge Messungen werden durchgeführt, wobei der Reagenslösung jeweils ein in der Tabelle 4 angegebenes Detergens in der dort angegebenen Konzentration zugesetzt worden ist. Die gefundenen Ergebnisse sind in Tabelle 4 zusammengefaßt.

### Tabelle 4

Reaktionsgeschwindigkeiten ($\Delta$E/min) der Peroxidase-Reaktion für Hämoglobin bzw. den Hämoglobin-Haptoglobin-Komplex mit Peroxid und Guajakol als Substrat in Gegenwart verschiedener Detergentien

| Guajakol (mmol/l) | Detergens (mg/ml) | $\Delta$E/min | | $\dfrac{\text{Hb : Hp}}{\text{Hb}}$ |
|---|---|---|---|---|
| | | Hb (mE/min) | Hb : Hp (mE/min) | Hb |
| 30 | — | 17 | 200 | 11,8 |
| 30 | 0,5 Saponin | 0 | 164 | >106 |
| 30 | 1,0 | 0 | 138 | >100 |
| 30 | 5 | 0 | 96 | >100 |
| 30 | 1,0 | 6[2] | 138 | 90 |
| 30 | 2,0 | 5,5[2] | 138 | 100 |
| 15 | — | 60 | 169 | 2,8 |
| 15 | 2,0 Saponin | 0 | 115 | >100 |
| 30 | — | 14,5 | 194 | 13,4 |
| 30 | 40 G2330[1] | 10 | 145 | 14,5 |
| 30 | 0,5 Digitonin | 3 | 154 | 50 |

[1] G 2330 = Polyoxyethylen-Sorbit-Detergens der Fa. Atlas
[2] Hämoglobin-Konzentration 1,00 mg Hb/ml

6

0 125 513

## Beispiel 5

Titration einer Haptoglobin-haltigen Probe mit Hämoglobin

Es wird eine Haptoglobin-Stammlösung in Wasser hergestellt. Aliquots dieser Stammlösung werden mit unterschiedlichen, genau bekannten Mengen Hämoglobin 10 min bei Raumtemperatur inkubiert. Proben der vorinkubierten Mischungen werden in gleiche Mengen einer Reagenslösung überführt, die 0,1 mol/l Natriumcitratpuffer, pH 4,0 und 0,5 mmol/l ABTS und 3 g/l Saponin enthält. Die Endkonzentration des Haptoglobins in der Küvette beträgt $0,64 \times 10^{-7}$ mol/l. Durch Zugabe von Natriumperborat-Lösung (Endkonzentration in der Küvette 5 mmol/l) wird die Peroxidase-Reaktion gestartet und die Reaktionsgeschwindigkeit über die Entstehung des grünen Farbstoffs gemessen (Wellenlänge 436 nm).

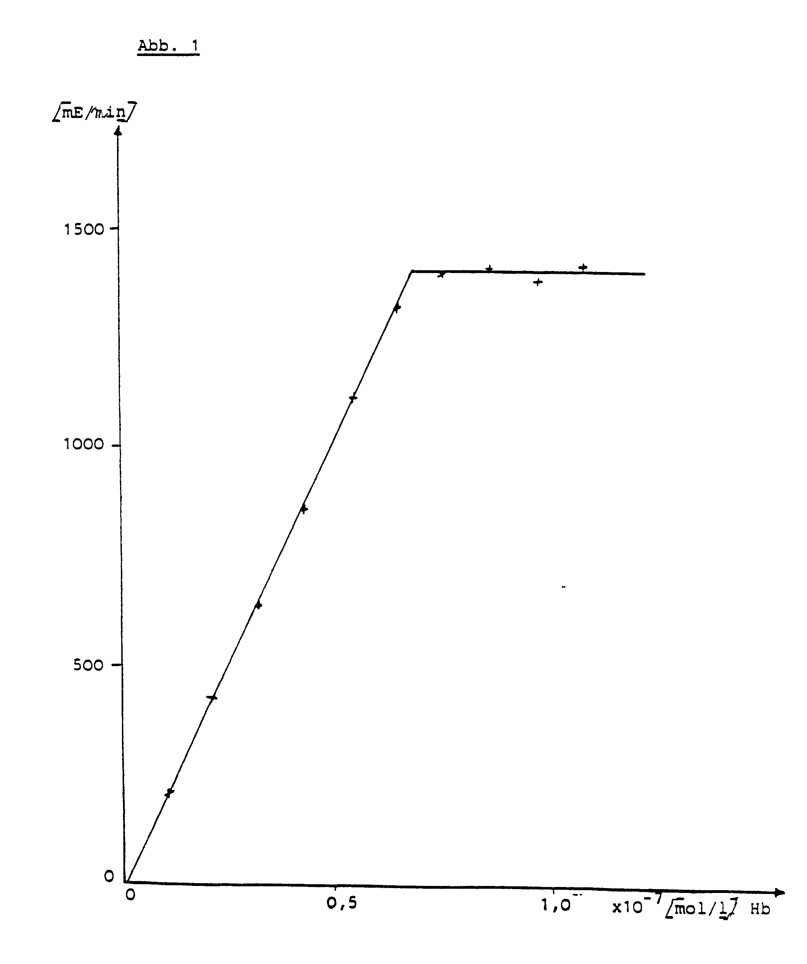

In Abbildung 1 ist die Reaktionsgeschwindigkeit in Abhängigkeit von der Hämoglobin-Konzentration im Reagens aufgetragen. Der Abbildung 1 ist zu entnehmen, daß die Reaktionsgeschwindigkeit linear mit zunehmender Hämoglobin-Konzentration ansteigt, bis das in der Lösung befindliche Haptoglobin vollständig als Hämoglobin-Haptoglobin-Komplex vorliegt. Weitere Hämoglobin-Zugabe über diese Sättigungskonzentration hinaus führt nicht mehr zu einer deutlich meßbaren höheren Reaktionsgeschwindigkeit.

## Beispiel 6

Bestimmung von glykosiliertem Hämoglobin

a) Verwendete Reagentien :

Reagens Ia : 50 mmol/l Bis-Tris-Puffer, pH 6,7
Reagens Ib : 50 mmol/l Bis-Tris-Puffer, pH 6,7
     0,08 mmol/l Inosithexaphosphat
Reagens II : 7 mmol/l Bis-Tris-Puffer, pH 6,7
    2 mmol/l ABTS
    20 mg/l Haptoglobin
    0,2 g/ml Saccharose
Reagens III : 0,5 mol/l Citratpuffer, pH 3,8
    30 mmol/l Natriumperborat
    3 g/l Saponin

b) Versuchsdurchführung

Von der zu messenden Probe Vollblut werden die Erythrozyten abzentrifugiert, dreimal mit physiologischer Kochsalzlösung gewaschen und durch Zugabe des gleichen Volumens Wasser hämolysiert. Das Hämolysat wird mit festem Natriumdithionit bis zur Konzentration 50 mg/ml und mit festem Natriumnitrit bis zur Konzentration 5 mg/ml versetzt. Nach einer kurzen Inkubation bei Raumtemperatur wird je ein Aliquot des vorbehandelten Hämolysats mit Reagens Ia bzw. Reagens Ib auf eine Hämoglobin-Konzentration von 0,5 mg/l eingestellt. Die so erhaltene Probelösung wird in die Probengefäße eines automatischen Analysegerätes, z. B. Abbott VP, eingefüllt. Das Gerät mischt jeweils 2,5 µl der Probelösung mit 2,5 µl Reagenslösung II. Es wird 40 Sekunden lang bei 25 °C inkubiert und danach die Peroxidasereaktion durch Zugabe von 150 µl Reagens III gestartet. Die Extinktionszunahme wird mit der Filterkombination 415/450 nm zwei Minuten lang verfolgt und die Reaktionsgeschwindigkeit als Quotient beobachte Extinktionsänderung/2 Minuten in mE/Min. ausgedruckt.

Die bei Zusatz von Reagens Ib zur Probe erhaltene Reaktionsgeschwindigkeit wird durch die mit Reagens Ia gemessene dividiert.

Durch analoge Messung und Auswertung von Standard-Proben bekannten Gehalts an glykosiliertem Hämoglobin wird eine Eichkurve erstellt, an der die Konzentration an glykosiliertem Hämoglobin in unbekannten Proben abgelesen werden können.

In Tabelle 5 sind die in der oben beschriebenen Weise ermittelten Werte für vier verschiedene Proben angegeben (Spalte 2). Zum Vergleich sind in Spalte 3 die entsprechenden Werte aufgeführt, die nach der bisher üblichen Methode durch Chromatographie an Haptoglobinhaltigen Ionenaustauschersäulen erhalten worden sind. Die Werte zeigen gute Übereinstimmung.

## Tabelle 5

Gehalt an glykosiliertem Hämoglobin (HbA$_1$) in verschiedenen Proben

A) gemessen nach Zusatz eines erfindungsgemäßen Detergens
B) gemessen durch Chromatographie an Ionenaustauschersäulen

7

| Probe Nr. | Methode A (%) HbA$_1$ | Methode B (%) HbA$_1$ |
|---|---|---|
| 1 | 7,5 | 7,3 |
| 2 | 16,4 | 16,0 |
| 3 | 8,8 | 8,9 |
| 4 | 11,2 | 12,9 |

**Patentansprüche**

1. Verfahren zur Bestimmung des Hämoglobin-Haptoglobin-Komplexes in Gegenwart von freiem Hämoglobin durch Ausnutzen der unterschiedlichen peroxidatischen Eigenschaften des freien und des gebundenen Hämoglobins, dadurch gekennzeichnet, daß zur selektiven Inhibierung der Peroxidase-Aktivität des freien Hämoglobins ein Detergens aus der Gruppe der Saponine, Lanoline und Detergentien mit einem Zuckeranteil zugesetzt und die restliche Peroxidase-Aktivität des Reaktionsgemisches gemessen wird.

2. Verfahren gemäß Anspruch 1, dadurch gekennzeichnet, daß das Detergens in einer Konzentration von 0,1 bis 50 g/l eingesetzt wird.

3. Verfahren gemäß einem der Ansprüche 1 und 2, dadurch gekennzeichnet, daß zum Messen der Peroxidase-Aktivität ein Peroxid und ein chromogenes Substrat verwendet wird.

4. Verfahren gemäß Anspruch 3, dadurch gekennzeichnet, daß als chromogenes Substrat Guajakol oder 2,2'-Azino-di-[3-äthylbenzthiazolinsulfonsäure(6)] eingesetzt wird.

5. Verfahren gemäß Anspruch 3, dadurch gekennzeichnet, daß als chromogenes Substrat 4-Aminoantipyrin, 3-Methyl-2-benzthiazolonhydrazon oder 3-Methyl-2-benzthiazolonhydrazonsulfonsäure in Kombination mit einem Phenol- oder Anilin-Derivat eingesetzt wird.

6. Reagenz zur Durchführung des Verfahrens gemäß einem der Ansprüche 1 bis 5, dadurch gekennzeichnet, daß es neben Substanzen zur Bestimmung der Peroxidase-Aktivität ein Detergens aus der Gruppe der Saponine, Lanoline und Detergentien mit einem Zuckeranteil zur Inhibierung der Peroxidase-Aktivität des freien Hämoglobins enthält.

7. Verfahren zur Bestimmung des Haptoglobin-Gehaltes einer Proben dadurch gekennzeichnet, daß die Probe mit einem Überschuß an Hämoglobin versetzt und der entstandene Haptoglobin-Hämoglobin-Komplex in Gegenwart des überschüssigen Hämoglobins mit Hilfe des Verfahrens gemäß einem der Ansprüche 1 bis 5 bestimmt wird.

8. Verfahren zur Bestimmung von glykosiliertem Hämoglobin in einer Probe durch Differenzierung von glykosiliertem und nicht-glykosiliertem Hämoglobin mit Hilfe von Haptoglobin und Messen der Peroxidase-Aktivität des glykosilierten Hämoglobin-Haptoglobin-Komplexes, dadurch gekennzeichnet, daß die Peroxidase-Aktivität des glykosilierten Hämoglobin-Haptoglobin-Komplexes in Gegenwart des freien Hämoglobins mit Hilfe des Verfahrens nach einem der Ansprüche 1 bis 5 bestimmt wird.

**Claims**

1. Process for the determination of the haemoglobin-haptoglobin complex in the presence of free haemoglobin by utilisation of the different peroxidate properties of the free and of the bound haemoglobin, characterised in that, for the selective inhibition of the peroxidase activity of the free haemoglobin, a detergent from the group of the saponins, lanolins and detergents with a sugar component is added thereto and the residual peroxidate activity of the reaction mixture is measured.

2. Process according to claim 1, characterised in that the detergent is used in a concentration of 0.1 to 50 g/l.

3. Process according to claims 1 and 2, characterised in that a peroxide and a chromogenic substrate is used for the measurement of the peroxidase activity.

4. Process according to claim 3, characterised in that guaiacol or 2,2'-azino-di-[3-ethyl-benzthiazoline-sulphonic acid(6)] is used as chromogenic substrate.

5. Process according to claim 3, characterised in that 4-aminoantipyrine, 3-methyl-2-benzthiazolone hydrazone or 3-methyl-2-benzthiazolone hydrazone-sulphonic acid, in combination with a phenol or aniline derivative, is used as the chromogenic substrate.

6. Reagent for the carrying out of the process according to one of claims 1 to 5, characterised in that, besides substances for the determination of the peroxidase activity, it contains a detergent from the group of the saponins, lanolins and detergents with a sugar component for the inhibition of the peroxidase activity of the free haemoglobin.

7. Process for the determination of the haptoglobin content of a sample, characterised in that the sample is mixed with an excess of haemoglobin and the resulting haptoglobin-haemoglobin complex is determined in the presence of the excess haemoglobin with the help of the process according to one of claims 1 to 5.

**0 125 513**

8. Process for the determination of glycosilated haemoglobin in a sample by differentiation of glycosilated and non-glycosilated haemoglobin with the help of haptoglobin and measurement of the peroxidase activity of the glycosilated haemoglobin-haptoglobin complex, characterised in that the peroxidase activity of the glycosilated haemoglobin-haptoglobin complex is determined in the presence of the free haemoglobin with the help of the process according to one of claims 1 to 5.

**Revendications**

1. Méthode pour la détermination du complexe hémoglobine-haptoglobine en présence d'hémoglobine libre en tirant parti des propriétés peroxydatiques différentes de l'hémoglobine libre et combinée, caractérisée par le fait que pour l'inhibition sélective de l'activité de peroxydase de l'hémoglobine libre, on ajoute un détergent du groupe des saponines, des lanolines et des détergents contenant une proportion de sucre et que l'on mesure l'activité résiduelle de peroxydase du mélange réactionnel.

2. Méthode selon la revendication 1, caractérisée par le fait que l'on utilise le détergent à une concentration de 0,1 à 50 g/l.

3. Méthode selon l'une des revendications 1 et 2, caractérisée par le fait que la mesure de l'activité de peroxydase, on utilise un peroxyde et un substrat chromogène.

4. Méthode selon la revendication 3, caractérisée par le fait que comme substrat chromogène, on utilise le guaiacol ou l'acide 2,2'-azino-bis-[3-éthylbenzothiazolinesulfonique(6)].

5. Méthode selon la revendication 3, caractérisée par le fait que comme substrat chromogène, on utilise la 4-aminoantipyrine, la 3-méthyl-2-benzothiazolone-hydrazone ou l'acide 3-méthyl-2-benzothiazolone-hydrazonesulfonique en association avec un dérivé de phénol ou d'aniline.

6. Réactif pour la mise œuvre de la méthode selon l'une des revendications 1 à 5, caractérisé par le fait qu'outre des substances pour la détermination de l'activité de peroxydase, il contient un détergent du groupe des saponines, des lanolines et des détergents contenant une proportion de sucre, pour l'inhibition de l'activité de peroxydase de l'hémoglobine libre.

7. Méthode pour la détermination de la teneur en haptoglobine d'un échantillon, caractérisée par le fait que l'on ajoute à l'échantillon un excès d'hémoglobine et que l'on détermine le complexe haptoglobine-hémoglobine formé en présence de l'hémoglobine en excès à l'aide de la méthode selon l'une des revendications 1 à 5.

8. Méthode pour la détermination de l'hémoglobine glycosilée dans un échantillon, par différenciation de l'hémoglobine glycosilée et non glycosilée à l'aide d'haptoglobine et mesure de l'activité de peroxydase du complexe glycosilé hémoglobine-haptoglobine, caractérisée par le fait que l'on détermine l'activité de peroxydase du complexe glycosilé hémoglobine-haptoglobine en présence de l'hémoglobine libre à l'aide de la méthode selon l'une des revendications 1 à 5.

Abb. 1